# EUROPEAN PATENT SPECIFICATION

(11) **EP 2 415 418 B1**
(45) Date of publication and mention of the grant of the patent: **27.11.2013**
(21) Application number: 11174862.0
(22) Date of filing: 21.07.2011
(51) Int. Cl.: A61B 19/00

(54) **Robotic surgical utensil**
Robotisches chirurgisches Utensil
Outil chirurgical robotique

(30) Priority: 04.08.2010 IT GE20100089
(43) Date of publication of application: 08.02.2012
(73) Proprietor: Surgica Robotica S.p.A., 34121 Trieste (IT)
(72) Inventor: Fiorini, Paolo, I-37131 Verona (IT); Reppele, Luca, I-37039 Tregnano, Province of Verona (IT); Morselli, Massimo, I-41038 San Felice Sul Panaro, Province of Modena (IT)
(74) Representative: Bergadano, Mirko

(56) References cited:
- WO-A2-2010/050771
- US-A- 5 784 542
- US-A- 5 797 900
- US-A1- 2007 088 340
- US-A1- 2008 262 654
- US-A1- 2010 004 663
- US-A1- 2010 011 901
- US-A1- 2010 082 041
- US-B1- 6 197 017

## Description

The present invention relates to a robotic surgical utensil for use, in particular, in minimally invasive surgical operations.

As is known, robotic surgical utensils are provided with an end member, referred to hereinbelow as "end effector", an instrument which is used to finish one or more phases of a minimally invasive surgical operation. This end effector may be formed, for example, by a tongs-like member comprising a pair of mobile arms.

US6394998B1 describes surgical utensils or instruments for use in minimally invasive surgical operations. These instruments are typically made up of a base, by means of which the utensil is mounted removably on a robotically controlled articulated arm. An elongate shaft extends from the base. One working end of the shaft is arranged distally with respect to the base. A wrist-like element provided with one or two degree(s) of freedom is mounted on this working end. At least one end effector is mounted rotatably on an opposite end of the wrist-like element. A plurality of elongate elements, for example cables, extend from the end effector and from the wrist-like element so as to realize defined angular movements of the wrist-like element and of the end effector in response to defined pulling actions of the elongate elements.

This end effector described in said document US6394998B1 comprises a pair of tongs-like members which can be rotated together or independently of one another by means of said elongate elements or activation cables.

The surgical utensil described in said document US6394998B1 has various disadvantages. Firstly, each of the activation cables of each of the members of the end effector follows the course of an endless ring: on one side of this endless ring, this cable wraps around a pulley part which is integral with the relative member of the end effector. Each of these ring-like cables is associated with an actuator, i.e. ultimately for each degree of freedom of the utensil there is an actuator for activating the end effector or a member thereof. This arrangement of the cables in an endless ring does not allow the forces active on each branch of the activation cable to be evaluated correctly. Another limitation of this arrangement of the known utensil in an endless ring as described above is that when the tool is removed from the robotic arm, the activation cables in an endless ring remain preloaded, i.e. they remain under tension both during normal use and (removed from the utensil) during the sterilization process. During the latter phase, the instrument is subjected to numerous treatments (e.g. thermal and cleaning treatments), which could tamper with the transmission cables and the preloading thereof.

US2010/082041A1 discloses a robotic surgical system using a passive preload system attached to a tendon that is wrapped around a capstan to control relaxed tension in the tendon. The passive preload system employs spring or other structure to apply tension to the tendon. The capstan is driven by a motor when the tendon is needed to pull on a structural member of the instrument (end effector). For example, for the application of clamping pressure or movement of the structural member against resistance, capstan friction can produce the tendon tension that is many times the tension applied by the passive preload system. However, when the tendon is not needed to apply force to the member, the capstan can be freed, so that the spring system provides enough tension to prevent derailment or other malfunctions of the tendon. The low tension in relaxed tendons can reduce tendon friction, particularly in instruments with flexible shafts.

The object of the present invention is therefore to provide a robotic surgical utensil which overcomes the disadvantages of known robotic surgical utensils, such as for example that described above, which can be used with increased proficiency and with high efficiency, allowing accurate measurement of the tension applied to each branch of the cable, which can be easily recognized by the user, which, once removed from the arm of the robotic control system to which it should be connected, allows immediate and accurate calibration every time it is reconnected to said arm, and which can therefore be subjected to an effective sterilization process without it being necessary to verify the preloading of the transmission cables. It is also plausible that the cable withstands more working cycles, since the thermal delta of about 100°C owing to the sterilization does not give rise to an increase in tension on the branches of the cable.

This object is achieved by the present invention by means of a robotic surgical utensil according to that specified in Claim 1.

Further advantageous features of the present utensil are presented in the dependent claims.

The present utensil is advantageously provided with a base for connection to the utensil holder, with an elongate shaft placed distally with respect to said base, with an articulated wrist having at least one degree of freedom, mounted on the working end of the elongate shaft, and with an end effector connected to the articulated wrist in a distal position. The transmission system can allow the actuation of the wrist and of the end effector, which, for example, can be pincers with two arms, in the case of the end effector according to at least one degree of freedom. Each degree of freedom is effected by means of a pair of actuators, which are positioned at the free ends of each of the elongate activation elements such that said elongate elements can be activated agonistically/antagonistically, which makes it possible to modify the impedance and control the tension applied to each branch of each flexible, elongate element.

In the transmission assembly, it is also advantageous that these flexible, elongate elements suitably cross around at least one pair of pulleys, allowing the decoupling of the axes of rotation of said pair of pulleys.

It is important to point out that the utensil is also provided with a connection and disconnection system for the flexible, elongate elements. At each disconnection end for the flexible elements, the system is provided with an elastic element for implementing effective control of the agonistic/antagonistic motion and for keeping the transmission elements under tension once the tool has been disconnected. This solution makes the processes for changing and sterilizing the utensil easier, allowing immediate calibration thereof at the end of these processes.

The present utensil also comprises a system for checking the correct positioning of the shaft in the interface support and simple and effective identification and recognition systems, such as a collar which can have various colourings depending on the type of utensil, a matrix of identifying stamps placed on the tool which is to be coupled as the latter is inserted into a receptive matrix on the utensil holder base, or a DataMatrix label, in which a series of serial data, of calibration data of the utensil and others are stored.

Further features and advantages of the present invention will be understood more clearly in the course of the following description, considered by way of example, no limitation being implied, and with reference to the appended drawings, in which:
- Figure 1 is a top view of a robotic surgical utensil according to the present invention, comprising a shaft to the distal end of which is connected an end effector and the proximal end of which is inserted into an interface support for connection to an arm of a robotic control and command system of the utensil;
- Figure 2 is a side view, on an enlarged scale, of the distal part of the shaft where a transmission assembly which allows relative motions of the end effector with respect to the shaft is positioned;
- Figure 3 is a top view, partially in section, of the transmission assembly of the utensil, in which a series of cables wrapped and crossed around three series of pulleys are provided;
- Figure 4 is a first side view, partially in section, of the transmission assembly of the utensil, in which it is possible to see the cables for actuating the members of the end effector and for realizing a yawing motion of the end effector with respect to the articulated wrist;
- Figure 5 is a second side view, partially in section, of the transmission assembly of the utensil, in which it is possible to see a pair of cables for realizing a pitching motion of the articulated wrist with respect to the shaft;
- Figure 6 is a third side view, partially in section, of the transmission assembly, which is provided with a pair of crossed support cables that define the kinematics and the dynamics of the pitching motion of the utensil;
- Figure 7 is a top view, in section, of the interface support of the present utensil;
- Figure 8 is a top view, in section, of an alternative embodiment of the interface support, wherein each of the branches of the actuation cables comprises a displacement demultiplication system positioned at the connection between the robotic arm and the tool;
   Figure 9 is a side view, partially in section, of the displacement demultiplication system of Figure 8; and
- Figure 10 is a top view of the displacement demultiplication system of Figures 8 and 9.

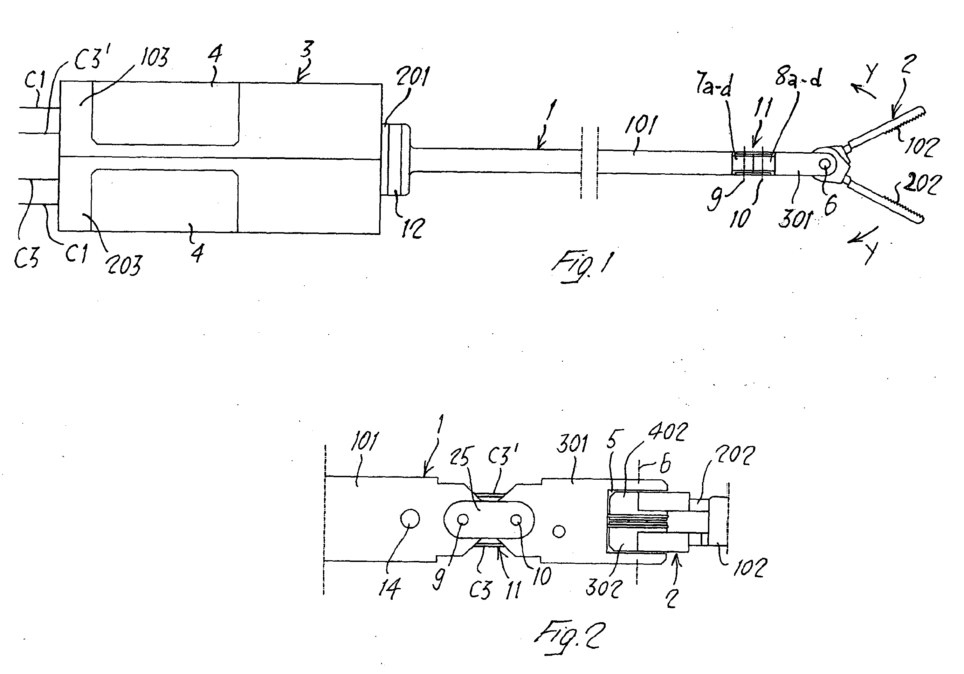

With reference to these appended drawings and with particular reference to Figure 1 therein, 1 denotes a substantially cylindrical shaft comprising a distal part 101, to the end of which is connected an end effector 2, in this case tongs with two arms 102 and 202, and a proximal part 201 connected to an interface support 3, that can be connected to an articulated arm (not shown) of a robotic control and command system of the utensil. This support 3 can be formed by two semicylindrical parts 103 and 203, which are hinged to one another at one side such as to open and close in order to receive the proximal part 201 of the shaft 1 and keep it in position, as illustrated in the figure, or may be formed by a single, internally hollow cylindrical element, into which the proximal part of the shaft can be inserted by means of a bayonet attachment or in any case attachment means which render the connection between this shaft 1 and this support 3 removable. Each of the two semicylindrical parts 103 and 203 of the support is provided with a removable cover 4 for inspecting and manipulating the various connections, which will be described below, present inside this support 3. The distal part 101 of the shaft 1 is connected to the end effector by means of a rotary pin 6 and comprises a transmission assembly 11, of which two series of pulleys 7a-d and 8a-d pivoted respectively on two pins 9 and 10 can be seen in the figure. This articulation assembly 11 is traversed by a series of flexible, elongate elements for actuating the end effector, for example cables, which also pass through the internally hollow shaft 1 and which will be described in detail below. The ends of each of said actuation cables project from the rear part of the utensil holder support 3; the figure shows the two free ends of the two branches of a first actuation cable C1 and the two rear, free ends of another two actuation cables C3 and C3'. Each of the arms 102 and 202 of the end effector 2, as can also be seen in Figure 2 of the appended drawings, is provided at the base with a substantially circular element, respectively the element 302 and the element 402, housed in a seat 5 made in the head 301 of the distal part 101 of the shaft 1, placed downstream of the transmission assembly 11. In this seat 5, the elements 302 and 402 overlap and engage on a rotary pin 6, which makes it possible for the end effector 2 to be imparted with an oscillatory yawing motion, in the directions of the arrows Y shown in Figure 1, if these elements 302, 402 and therefore the corresponding arms 102, 202 are rotated together in the same direction in one direction or the other. A collar 12 for identifying the present utensil is positioned on the proximal part 201 of the shaft 1. This collar 12 may be realized in various colourings for each type of utensil, such that it can be recognized quickly by the user. The present utensil is also provided with a further recognition and identification system based, for example, on a matrix of coupling stamps and on a DataMatrix label, which is positioned in any part of the utensil, for example on the shaft 1. These systems can contain a series of data, such as the serial identification data of the utensil, the calibration data thereof and others, which are read and inserted into the robotic command and control system to which a connection is made.

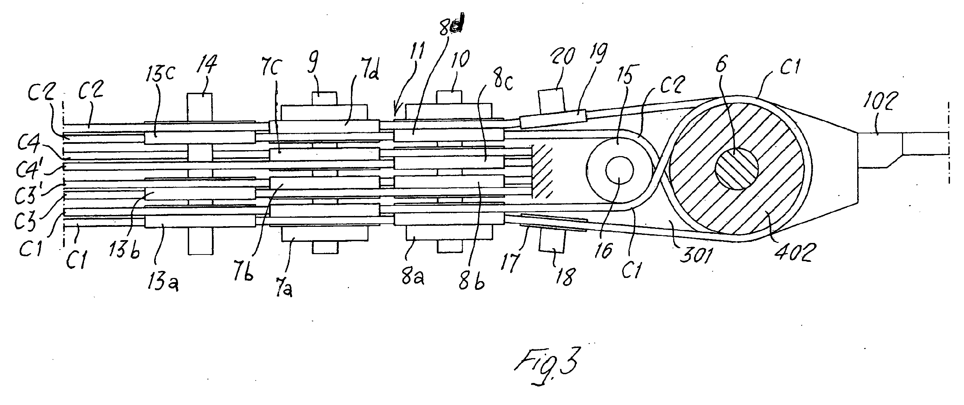

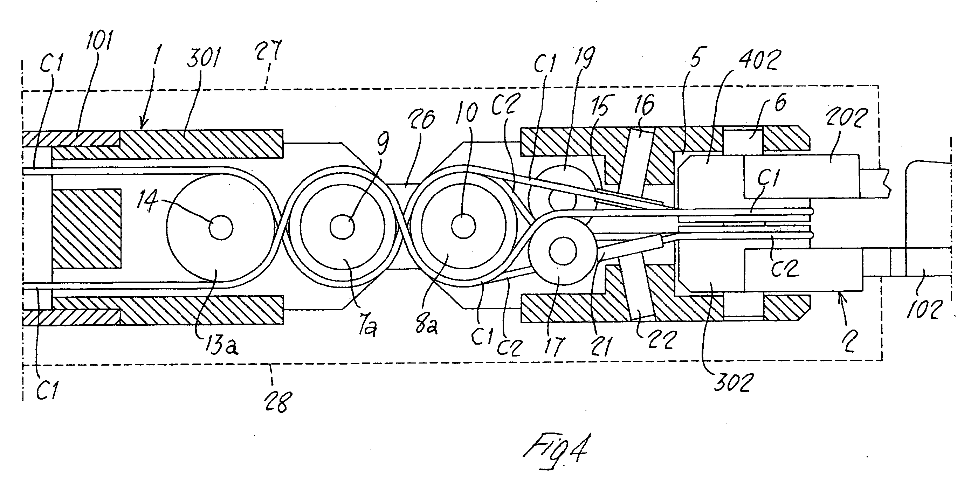

Figure 3 shows the transmission assembly 11 with the crossed cables for actuating and supporting the present utensil. This group 11 comprises three series of adjacent pulleys: a first series of pulleys 13a, 13b and 13c which are placed a certain distance apart and are freely rotatable around a relative pin 14; a second series of pulleys 7a, 7b, 7c, 7d which are placed a certain distance apart and are freely rotatable around a relative pin 9; and a third series of pulleys 8a, 8b, 8c, 8d which are placed a certain distance apart and are freely rotatable around a relative rotary pin 10. These rotary pins 14, 9 and 10 of the three series of pulleys are parallel to one another. The following elements are present in said transmission assembly 11: the first activation cable C1 of one of the two arms of the end effector; a second activation cable C2 of the other arm of the end effector; the pair of cables C3 and C3' which can realize the pitching motion of the end effector; and a pair of support cables C4 and C4'. Each of said activation and support cables C1, C2, C3, C3', C4 and C4' is suitably crossed around said three series of pulleys 13a-c, 7a-d and 8a-d. The first cable C1 can activate the arm 202 of the end effector, which cannot be seen in the figure and is integral with the circular element 402 shown in section. This first cable C1 performs a round course in contact with the upper or lower part of the pulleys 13a, 7a and 8a of the first series of pulleys. More specifically, this cable C1 passes over the first pulley 13a of the first series of pulleys, then under the first pulley 7a of the second series, over the first pulley 8a of the third series of pulleys, turns around a further pulley 15, which is freely rotatable around a relative rotary pin 16, then turns around the circular element 402 integral with the arm 202, passes over a further pulley 17 with an inclined rotary pin 18, passes under the pulley 8a, over the pulley 7a and finally under the pulley 13a. An appropriate circumferential channel may be made in the circular element 402, which is covered by a stretch of the cable C1, in order to guarantee better contact and fixing of this stretch around this element 402. Ultimately, therefore, the two branches of the cable C1 are doubly crossed around the pulleys 13a, 7a, 8a. Like the cable C1, the second cable C2 comprises two free ends projecting from the rear of the transmission assembly 11. This cable C2 performs a course around the aligned pulleys 13c, 7d and 8d which is similar to the course performed by the cable C1 around the first aligned pulleys 13a, 7a and 8a. This cable C2, after it has passed through these pulleys 13c, 7d and 8d by crossing them for the first time, passes around a return pulley 21, which can be seen in Figure 4 and is provided with a relative rotary pin 22, in a position which is symmetrical with respect to the position in which the cable C1 passes around the pulley 15, then turns around the circular element 302, which cannot be seen in the figure and is integral with the arm 102 of the end effector. An appropriate circumferential channel may also be made in the circular element 302, which is covered by a stretch of the cable C2, in order to guarantee better contact and fixing of this stretch around this element 302. After it has turned around this circular element of the arm 102, this cable C2 passes around a further pulley 19, which has an inclined rotary pin 20 and is situated in a position which is symmetrical with respect to the pulley 18 with respect to a longitudinal mid-plane of the utensil, and then passes over the pulley 8d, under the pulley 7d and finally over the pulley 13c. Ultimately, therefore, the two branches of the cable C2 are doubly crossed around the pulleys 13c, 7d and 8d. As mentioned, the two cables C3 and C3' can execute the pitching motion of the end effector 2 with respect to the distal part 101 of the shaft 1. The cable C3 passes under the pulley 13b, over both the pulleys 7b and 8b, and then the front end thereof is fixed, as can be seen, to the head 301 of the shaft. The cable C3', in a position crossed with respect to the cable C3, passes over the pulley 13b, under both the pulleys 7b and 8b, and then the front end thereof is fixed to the head 301. The two cables C4 and C4' are the only cables not to be connected to relative actuators: the cable C4 passes under the pulley 7c, over the pulley 8c, and then the front end thereof is fixed to the head 301; the cable C4', in a position crossed with respect to the cable C4, passes over the pulley 7c, under the pulley 8c, and the front end thereof is fixed to the head 301.

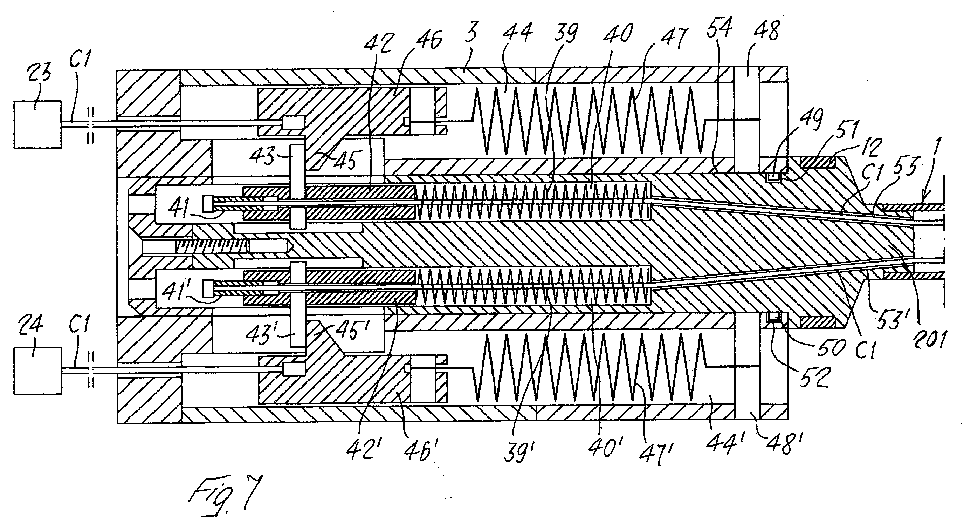

The two ends of each of these cables C1, C2 and the front ends of the two cables C3, C3' are free and are each connected to their own actuator; by way of example, Figure 7 shows the two activation actuators 23 and 24 which are connected to each of the two free ends of the cable C1 and can activate the two branches of this cable C1 projecting from the interface support 3 independently and agonistically/antagonistically. These actuators 23 and 24 may be located, for example, in an arm of the robotic system which commands and operates the present surgical utensil. In substance, six actuators are therefore provided for the present utensil: two actuators connected to the free ends of the cable C1, two actuators connected to the free ends of the cable C2 and two actuators connected to the rear ends of the cables C3 and C3'.

The section in Figure 4 provides a better explanation of the arrangement of the activation cable C1 of the arm 202 of the end effector 2, in particular the crossing of the two branches of this cable around the three pulleys 13a, 7a, 8a, until it is wrapped and fixed around the circular element 402 integral with the arm 202 of the end effector. In this figure, it is also possible to see part of the cable C2, which is fixed around the circular element 302 integral with the arm 102 of the tongs. As can be seen, the rotary pin 6 of the two circular elements 302 and 402 is perpendicular to the three rotary pins 14, 9 and 10 of the three series of pulleys, of which the first three pulleys 13a, 7a, 8a can be seen in the figure, whereas the two return pulleys 21 and 15 of the two cables C2 and C1 are in an opposite position with respect to a longitudinal mid-plane of the utensil passing through the three pins 14, 9, 10. The two rotary pins 9 and 10 of the pulleys 7a and 8a are fixed at both ends to a pair of lateral connection strips: the present figure shows the rear strip 26, whereas the front strip 25 can be seen in figure 2. The rotary pin 14 of the pulley 13a is fixed to the walls of the rear part of the head 301 inserted into the distal part 101 of the shaft 1, whereas the pins 6, 16, 22, 18 and 20 are fixed in suitable seats made on the front part of the head 301. The figure also shows, as dashes, two electrification cables 27 and 28 of the arm 202 and of the arm 102, respectively, of the end effector 2. These cables 27 and 28 are connected upstream by means of standard connectors provided in an appropriate medical apparatus.

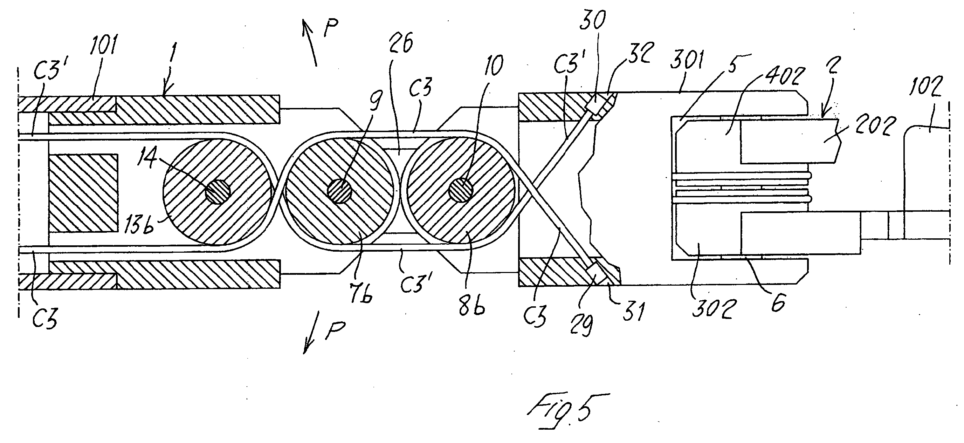

Figure 5 shows the cables C3 and C3', which make it possible to realize the pitching motion of the wrist 301 with respect to the distal part of the shaft 1, i.e. an oscillatory motion in the direction of the arrows P performed with respect to the pin 10, which rotates in turn around the axle 9 according to a set geometrical relationship. As mentioned above, these two cables C3 and C3' cross around the three pulleys 13b, 7b and 8b and the respective front ends are fixed to the head 301 of the shaft 1 by means of relative fixing elements 29 and 30 inserted removably into relative seats 31 and 32 made in said head 301.

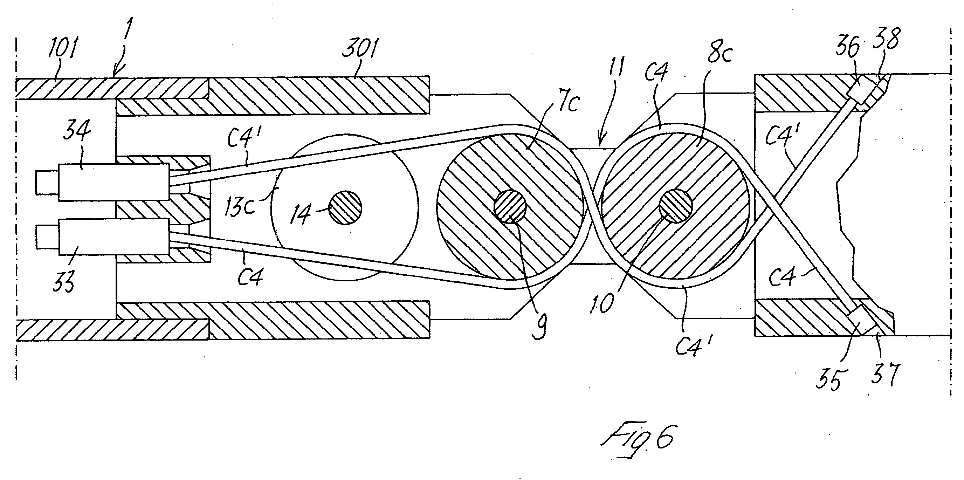

Figure 6 shows the two cables C4 and C4', the rear ends of which are connected to respective tensioning means 33 and 34 housed in the rear part of the head 301 inserted into the distal part 101 of the shaft 1. The function of these cables C4 and C4' is to support the articulation assembly 11 of the present utensil and these cables define the kinematics and the dynamics of the pitching motion described in Figure 5. These cables C4 and C4' cross around the two pulleys 7c and 8c and the respective front ends are fixed to the front part of the head 301 by means of relative fixing elements 35 and 36 inserted removably in relative seats 37 and 38 made on the front part of said head 301.

Figure 7 shows a further section of the utensil and of the utensil holder base, which highlights the disconnections of the two branches of the actuation cable C1 from within the shaft 1, in the cylindrical interface support 3, and therefore the emergence of the two free ends from the rear part of this support 3 and the connection thereof to the relative actuator 23 and 24. The elements described below for the two branches of the cable C1 are provided in exactly the same manner for the two branches of the cable C2 and for the two cables C3 and C3', i.e. for all the actuation cables of the present utensil which project from the rear part of the support 3 and the rear, free ends of which are provided with a relative actuator. The two branches of the cable C1 coming from within the shaft 1 pass through two channels 53 and 53' made in the proximal part 201 inserted into a cylindrical seat 54 made within the support 3. Each of the branches of the cable C1, see the upper branch for example, passes through a spring 39, for pretensioning the cable, housed in a cylindrical seat 40 made in the base of the instrument 201, and it is then connected by means of a crimp provided with extensometers 41 to a first slider 42 that can slide bidirectionally in said seat 40. The function of each of these sensorized crimps 41 and 41' is to measure the force applied to each of the branches of the cable C1, which are actuated agonistically/antagonistically. As an alternative to that shown, these extensometers may be positioned next to the end effector 2. Each of the two branches of the cable C1 is then interrupted next to said slider 42 or 42'. Each of these sliders 42 and 42', consider for example the slider 42, is provided with a pin 43 which is substantially perpendicular to the latter and engages on a corresponding tab 45 of a second slider 46, that can slide bidirectionally within a cylindrical seat 44 made near the peripheral part of the support 3. The last stretches of the two branches of the cable C1 are fixed to the rear of these second sliders 46 and 46', the free ends of these branches, as mentioned, being connected to a relative actuator 23 and 24 housed, for example, in the robotic command and control system of the utensil. Ultimately, even if the actuation cable C1 is interrupted by means of the connection and disconnection system represented by the two sliders 42, 46 and 42', 46', this cable C1, during operation of the utensil, is like a continuous cable with two branches, and the function of this interruption is advantageously to permit the disconnection of this actuation cable and therefore ultimately the disconnection of the shaft 1 from the support 3. One end of a tensile spring 47 is fixed to the front part of each of these second sliders 46 and 46', consider for example the slider 46, and the other end of said tensile spring is connected to the front base 48 of the cylindrical seat 44. In substance, the function of the two springs 39 and 39' associated with the two sliders 42 and 42' is to push and pretension each of the branches of the cable C1 which move towards the end effector, so as to always subject these branches to tension, even if the proximal part 201 of the utensil is not inserted in the support 3.

The two springs 47 and 47' associated with the two sliders 46 and 46' also have the function of keeping the two branches of the actuation cable C1 under tension and, once the instrument is connected, of opposing the actuators 23 and/or 23' when they pull the slider 46 and/or 46' towards the rear part of the support. The present utensil is also provided with a system for automatically recognizing the correct insertion thereof into the support 3. This system is based on mechanical protrusions positioned on the instrument, which activate (with various combinations) electrical or optical signals on the instrument holder base. The figure shows, by way of example, two teeth 49 and 50, which protrude respectively from the cylindrical seat 54 of the support and from the proximal part 201 of the shaft 1 and which, if this shaft 1 is positioned correctly in the support 3, formed in this case by the two semicylindrical parts 103 and 203 of figure 1, engage in corresponding recesses 51 and 52 made respectively in the proximal part 201 and in the cylindrical seat 38. Electrical contacts may also be present in these recesses. This system for automatically recognizing the correct insertion of the utensil may be different if, for example, the support 3 is formed by a single cylindrical piece in which a cylindrical seat is made internally, and the proximal part of the shaft is inserted, for example by means of a bayonet attachment, inside this cylindrical support seat.

As mentioned above, the elements described above for the two branches of the cable C1 are also provided for the two branches of the cable C2 and the two actuation cables C3 and C3', i.e. each of these branches and cables comprises a disconnection and activation system formed by pairs of first and second sliders like those described for the cable C1, associated with relative pretensioning and thrust springs. These two branches of the cable C2 and the two cables 3 and 3' reach from within the shaft 1 and are inserted into relative channels, similar to the channels 53 and 53' of the branches of the cable C1, and move towards said disconnection and activation system. It is preferable for the six channels traversed by the cables C1, C2, C3 and C3', the six seats of the springs and of the first sliders to be made in the proximal part 201 of the head 1 such that they are at equal distances apart and are distributed uniformly in said proximal part 201, and also for the six seats of the springs and of the second sliders to be made in the body of the support 3 such that they are distributed uniformly and at equal distances apart in said support.

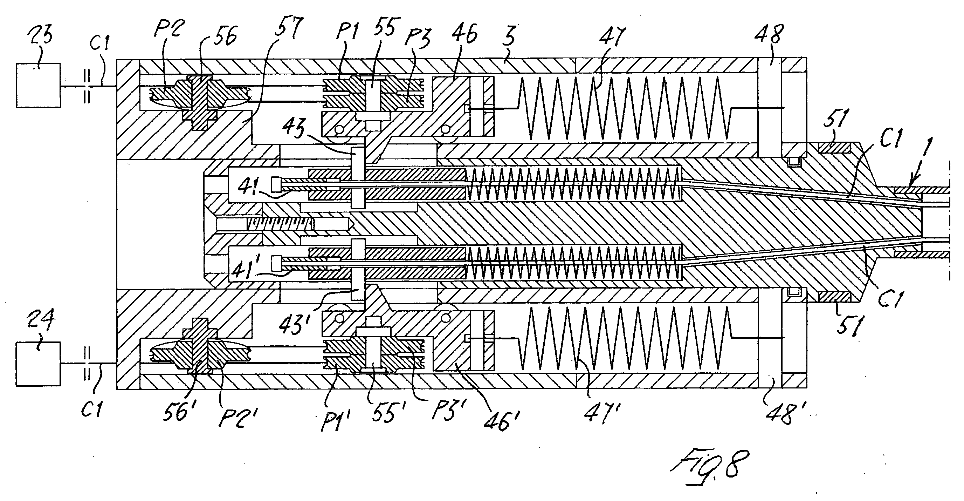

Fig. 8 shows an alternative embodiment of interface support 3, wherein each of the branches of the actuation cable C1, connected to the relative actuators 23 e 24, comprises a displacement demultiplication system. The elements described below for the two branches of the cable C1 are provided in exactly the same manner for the two branches of the cable C2 and for the two cables C3 and C3', i.e. for all the actuation cables of the present utensil which project from the rear part of the support 3 and the rear, free ends of which are provided with a relative actuator. The displacement demultiplication system comprises one or more rotating elements, for example pulleys, operating as a kind of simple or multiple hoist. By way of not limiting example, three pulleys P1-P1', P2-P2' and P3-P3' are shown in figure for each of the branches of the actuatione cable C1. Considering for example the superior branch of the actuation cable C1, the first pulley P1 is arranged on a first rotation pin 55 fixed on the slider 46, the second pulley P2 is arranged on a second rotation pin 56 connected to a fixed part 56 of the support 3 and the third pulley P3 is arranged on the first rotation pin 55, coaxially to the the first pulley P1.

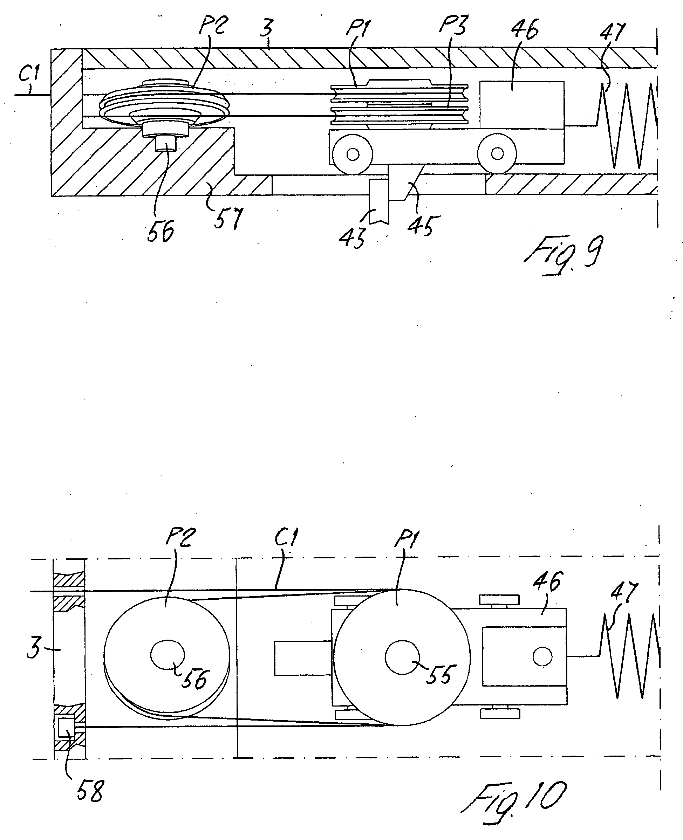

The branch of the cable C1 connected to the actuator 23, see Figures 9 and 10, is wrapped around the first pulley P1, around the second pulley P2, around the third pulley P3 and then fixed to the support 3 by means of a relative anchorage element 58.

Such a displacement demultiplication system confers a better accuracy and sensitivity to the present utensil, in fact a certain displacement of the branch of the cable C1 from the side of the actuators 23 and 24 produces a corresponding demultiplicated displacement of the branch of the cable C1 from the side of the end effector. The degree of said displacement demultiplication depends on the dimension, inclination and numbers of the pulleys of the system and on the wrapping path of the actuation cable around said pulleys. The pulleys used in the present system are however are realized in such a way to maintain the two branches of the actuation cable parallel to each other, thus obtaining a linear law of the demultiplicated displacement.

On account of the actuation of each of the branches of the cables C1 and C2 and the actuation of the two cables C3 and C3' by a dedicated actuator, i.e. in total six actuators provided, the present utensil ultimately has three degrees of freedom, i.e. every degree of freedom is actuated by means of two actuators. The provision of a dedicated actuator for each of the ends of the cables C1 and C2 and the provision of a dedicated actuator and an elastic element at each rear end of the cables C3 and C3' make it possible to activate each of the degrees of freedom agonistically/antagonistically, while also making it possible, by means of a relative system of extensometers to which each cable or branch of the cable is connected (see the two sensorized crimps 41 and 41' of Figure 7 for example), to control the impedance of the utensil on account of the measurement of the tension of each branch of the cables C1 and C2 and of each cable C3 and C3'.

As seen above, all the activation and support cables present in the transmission assembly 11, i.e. the cables C1, C2, C3 and C3' and the two cables C4 and C4', also all cross around at least one pair of pulleys, and this arrangement has many advantages, including the fact that it permits increased proficiency of the use of the present utensil and the decoupling of the axes of rotation of the pulleys around which these cables cross.

The operation of the present utensil according to that described in detail above is readily obvious. The two arms 102 and 202 of the end effector 2 can be moved independently of one another, as mentioned, by the respective agonistic/antagonistic activation of each of the ends of the cables C2 and C1. Therefore, if the circular elements 302 and 402 respectively integral with these arms 102 and 202 are rotated in the same direction of rotation, this results in the yawing motion of the end effector 2, which rotates around the axle 6 in one direction or the other (see the arrows Y of Figure 1). If, however, these circular elements 302 and 402 are rotated, always around the axle 6, in an opposite direction of rotation, the result is that the arms 102 and 302 of the end effector 2 move away from or towards one another, executing a typical tongs-like opening and closing motion. The end effector 2 may also be inclined in different ways by means of the pitching motion around the axle 10, which rotates in turn around the axle 9 according to a set geometrical relationship (see the arrows P of Figure 5), and this motion is completed by means of the activation of the pair of cables C3 and C3'. Each of the six actuators for activating each branch of the cables C1 and C2 and each cable C3 and C3' (see for example in Figure 7 the two actuators 23 and 24 for activating each of the branches of the cable C1) can be for example, an electric motor or any other means known per so which makes bidirectional tanslation of each of these branches of these cables C1 and C2 and of each of these cables C3 and C3' possible independently of one another and agonistically/antagonistically.

## Claims

1. Robotic surgical utensil, comprising: a shaft (1) bearing a distal part (101) and a proximal part (201); an articulated wrist (301) with at least one degree of freedom with respect to said shaft (1); an end effector (2), which is mounted on the front part of the wrist (301) and is provided with mobile arms (102, 202); an interface support (3), which is connected to said proximal part (201) and is connectable to a robotic command and control system of the utensil; and a transmission assembly (11) positioned in said articulated wrist (301) and comprising a plurality of pulleys (13a-c, 7a-d, 8a-d) positioned in sequence and a plurality of flexible, elongate activation elements (C1, C2, C3, C3') passing around the pulleys (13a-13c, 7a-7d, 8a-8d) so as to allow the movement of the wrist (301) according to said at least one degree of freedom and/or the movement of at least one of said mobile arms (102, 202); each of said flexible, elongate activation elements (C1, C2, C3, C3') cooperating, downstream of said transmission assembly (11), with said end effector (2) and with the relative mobile arms (102, 202), being provided, upstream of said transmission assembly (11), with at least one pair of free ends connectable to two actuators (23, 24) in such a way that said actuators (23, 24) can activate said flexible, elongate activation elements (C1, C2, C3, C3') independently of one another and agonistically/antagonistically to carry out said movement according to said at least one degree of freedom, the utensil being **characterized in that**: the wrist has a rear part connected to said distal part (101) and a front part movably connected to the rear part by means of two lateral connection strips (25, 26) to provide the wrist (301) with said at least one degree of freedom; the elongate activation elements (C1, C2, C3, C3') are crossed around at least one pair of pulleys (13a-13c, 7a-7d, 8a-8d) in a fashion that decouples the axes of rotation of the pulleys around which these elongate elements cross; said plurality of pulleys (13a-c, 7a-d, 8a-d) comprise at least three series of pulleys (13a-13c, 7a-7d, 8a-8d) arranged to freely rotate on three respective rotary pins (14, 9, 10) substantially parallel to one another; said pins (14, 9, 10) comprise a first pin (14) fixed to said rear part of the wirst (301) for the rotation of a first series of pulley (13a-13c), a second pin (9) fixed to first ends of both lateral connection strips (25, 26) for the rotation of a second series of pulleys (7a-7d), and a third pin (10) fixed to the second ends of both lateral connection strips (25, 26) for the rotation of the third series of pulleys (8a-8d); said flexible, elongate activation elements (C1, C2, C3, C3') comprise a first flexible, elongate element (C1) fixed, downstream of the transmission assembly (11), around a respective circular element (402) integral with a first mobile arm (202) of the end effector (2) and bearing, upstream of the transmission assembly (11), two first branches terminating, each, with a respective one of said free ends; and the two first branches are crossed a first time around and between a first pulley (13a) of said first series of pulleys (13a-13c) and a first pulley (7a) of said second series of pulleys (7a-7d) and a second time around and between the first pulley (7a) of the second series of pulleys (7a-7d) and a first pulley (8a) of said third series of pulleys (8a-8d).

2. Utensil according to Claim 1, **characterized in that** said flexible, elongate activation elements (C1, C2, C3, C3') comprise a second flexible, elongate element (C2) fixed, downstream of the transmission assembly (11), around respective circular element (302) integral with a second mobile arm (202) of the end effector (2) and bearing, upstream of the transmission assembly (11), two second branches terminating, each, with a respective one of said free ends; the two second branches are crossed a first time around and between a second pulley (13c) of said first series of pulleys (13a-13c) and a second pulley (7d) of said second series of pulley (7a-7d) and a second time around and between the second pulley (7d) of the second series of pulleys (7a-7d) and a second pulley (8d) of said third series of pulleys (8a-8d).

3. Utensil according to Claims 1 and 2, **characterized in.that** said first and second flexible, elongate elements (C1, C2) can impart the end effector (2) with a yawing motion (Y) with respect to a rotary pin (6), around which said arms (102, 202) of the end effector (2) can rotate.

4. Utensil according to Claim 1, **characterized in that** said flexible, elongate activation elements (C1, C2, C3, C3') comprise a pair of third flexible, elongate elements (C3, C3') which are connected, downstream of the transmission assembly (11), to said front part of the wrist (301) and are provided, upstream of the transmission assembly (11), with a respective free ends connected to a relative actuator, said pair of third flexible, elongate elements (C3, C3') being able to perform, by means of the relative actuator, a pitching motion (P) of the end effector (2) with respect to the distal part (101) of the shaft.

5. Utensil according to Claim 4, **characterized in that** said pair of third flexible, elongate elements (C3, C3') are crossed a first time around and between a third pulley (13b) of said first series of pulleys (13a-13c) and a third pulley (7b) of said second series of pulleys (7a-7d) and a second time around a third pulley (8b) of said third series of pulleys (8a-8d), said pitching motion (P) being performed with respect to said third pin (10), which rotates in turn around said second pin (9) according to a set geometrical relationship.

6. Utensil according to Claim 1, **characterized in that** said transmission assembly (11) comprises a pair of flexible, elongate support elements (C4, C4') for providing the transmission assembly (11) with a support and defining the kinematics and the dynamics of the pitching motion (P) of the end effector (2) with respect to the distal part (101) of the shaft (1); said flexible, elongate support elements (C4, C4') comprising respective rear ends connected to tensioning means (33, 34) housed in said rear part of the wrist (301) and respective front ends fixed to said front part of the wrist (301) by means of respective fixing elements (35, 36) removably inserted in respective seats (37, 38) made on the front part of the wrist (301).

7. Utensil according to Claim 6, **characterized in that** said flexible, elongate support elements (C4, C4') are crossed around and between a fourth pulley (7c) of said second series of pulleys (7a-7d) and a fourth pulley (8c) of said third series of pulleys (8a-8d).

8. Utensil according to Claim 1, **characterized in that** it comprises a connection and disconnection system positioned in said interface support (3) for connecting and disconnecting said flexible, elongate activation elements (C1, C2, C3, C3') and for connecting and disconnecting the interface support (3) to and from the shaft (1); the connection and disconnection system being provided with a first slider (42, 42'), which can slide in a relative first seat (40, 40') made in the proximal part (201) o:f the shaft (1) and can engage, by means of a relative connection element (43, 43'), with a second slider (46, 46') which can slide in a relative second seat (44, 44') made in the interface support (3).

9. Utensil according to Claim 8, **characterized in that** first elastic pretensioning means (39, 39') for said branches of said first flexible, elongate element (C1), positioned in said first seat (40, 40') of the proximal part (101) of the shaft (1).

10. Utensil according to Claim 8, **characterized in that** second elastic pretensioning means (47, 47') for said branches of said first flexible, elongate element (C1), positioned in said second seat (44, 44') of the interface support (3).

11. Utensil according to Claim 1, **characterized in that** it comprises measuring means (41, 41') for measuring the tension of each branch of said first and second flexible, elongate elements (C1, C2) or of each third flexible, elongate elements (C3, C3').

12. Utensil according to Claims 10 and 11, **characterized in that** said measuring means (41, 41') are arranged alongside said connection and disconnection system for each branch of said first and second flexible, elongate elements (C1) or for each third flexible, elongate element (C3, C3').

13. Utensil according to Claim 1, **characterized in that** it comprises at least one identification collar (12).

14. Utensil according to Claim 1, **characterized in that** it comprises a recognition and identification system based on a DataMatrix label, which is to be positioned in any part of the utensil and can contain a series of data, such as the serial identification data of the utensil, the calibration data thereof and others.

15. Utensil according to Claim 1, **characterized in that** it comprises a recognition system (49, 50, 51, 52) for automatically recognizing the correct insertion of the proximal part (201) of the shaft (1) in the interface support (3), said system being based on a mechanical connection on the part of the utensil, which, in accordance with the interface support (3), activates an electrical or optical signal or a signal based on a combination of said types.

16. Utensil according to Claim 1, **characterized in that** each of the mobile arms (102, 202) of the end effector (2) is provided with an electrical connection (27, 28), by means of which it can be connected to a standard connector of an appropriate medical apparatus.

17. Utensil according to Claim 1, **characterized in that** it comprises a displacement demultiplication system for each of the flexible, elongate activation elements (C1, C2, C3, C3'), said displacement demultiplication system comprising rotating elements (P1-P1', P2-P2', P3-P3') around which said flexible, elongate activation elements (C1, C2, C3, C3') are wrapped; said displacement demultiplication system comprising at least a fifth pulley (P1, P1') fixed to a movable part (46) of the utensil and at least a sixth pulley (P2, P2') connected to a fixed part (57) of the utensil.

18. Utensil according to Claim 17 when dependent on claim 8, **characterized in that** said fifth pulley (P1, P1') is connected to said second slider (46, 46') by means of a first rotation pin (55) and said sixth pulley (P2, P2') is connected to the fixed part (57) by means of a second rotation pin (56).

19. Utensil according to Claim 17, **characterized in that** said rotating elements (P1-P1', P2-P2', P3-P3') are realized in such a way to maintain the branches of the flexible, elongate activation elements (C1, C2, C3, C3') parallel to each other, thus obtaining a linear law of the demultiplicated displacement.

## Patentansprüche

1. Robotisches chirurgisches Instrument mit: einer Welle (1), die einen distalen Teil (101) und einen proximalen Teil (201) trägt; einem angelenkten Gelenkteil (301) mit mindestens einem Freiheitsgrad relativ zu der Welle (1); einem End-Effektor (2), der an dem vorderen Teil des Gelenkteils (301) angeordnet ist und mit bewegbaren Armen (102, 202) versehen ist; einer Zwischenverbindungs-Halterung (3), die mit dem proximalen Teil (201) verbunden ist und mit einem robotischen Befehls- und Steuerungssystem des Instruments verbindbar ist; und eine Übertragungsanordnung (11), die in dem angelenkten Gelenkteil (301) positioniert ist und mehrere hintereinander angeordnete Riemenscheiben (13a-c, 7a-d, 8a-d) und mehrere flexible, längliche Aktivierungselemente (C1,C2,C3,C3') aufweist, die um die Riemenscheiben (13a-c, 7a-d, 8a-d) laufen, um die Bewegung des Gelenkteils (301) entsprechend dem mindestens einen Freiheitsgrad und/oder der Bewegung mindestens eines der bewegbaren Arme (102, 202) zu ermöglichen; wobei jedes der flexiblen, länglichen Aktivierungselemente (C1,C2,C3, C3') die stromabwärts der Übertragungsanordnung (11) mit dem End-Effektor (2) und mit den betreffenden bewegbaren Armen (102,202) zusammenarbeiten, stromaufwärts der Übertragungsanordnung (11) mit mindestens einem Paar freier Enden versehen ist, die derart mit zwei Aktuatoren (23,24) verbindbar sind, dass die Aktuatoren (23,24) die flexiblen, länglichen Aktivierungselemente (C1, C2,C3, C3') unabhängig voneinander und agonistisch/antagonistisch aktivieren können, um die Bewegung entsprechend dem mindestens einen Freiheitsgrad auszuführen, wobei das Instrument **dadurch gekennzeichnet ist, dass**: das Gelenkteil einen hinteren Teil, der mit dem distalen Teil (101) verbunden ist, und einen vorderen Teil hat, der mittels zweier seitlicher Verbindungsstreifen (25,26) bewegbar mit dem hinteren Teil verbunden ist, um das Gelenkteil (301) mit mindestens einem Freiheitsgrad zu versehen; wobei die länglichen Aktivierungselemente (C1,C2,C3,C3') um mindestens ein Paar von Riemenscheiben (13a-c, 7a-d, 8a-d) derart kreuzweise verlaufen, dass die Drehachsen der Riemenscheiben, um die diese länglichen Elemente kreuzweise verlaufen, entkoppelt sind; wobei die mehreren Riemenscheiben (13a-c, 7a-d, 8a-d) mindestens drei Reihen von Riemenscheiben (13a-c, 7a-d, 8a-d) aufweisen, die zur freien Drehung an jeweiligen Drehstiften (14,9, 10) angeordnet sind, welche im Wesentlichen parallel zueinander angeordnet sind; wobei die Stifte (14,9,10) einen ersten Stift (14), der zwecks Drehung einer ersten Reihe von Riemenscheiben (13a-c) an dem hinteren Teil des Gelenkteils (301) angeordnet ist, einen zweiten Stift (9), der zwecks Drehung einer zweiten Reihe von Riemenscheiben (7a-d) an ersten Enden beider seitlicher Verbindungsstreifen (25,26) befestigt ist; und einen dritten Stift (10) aufweisen, der zwecks Drehung der dritten Reihe von Riemenscheiben (8a-d) an zweiten Enden beider seitlicher Verbindungsstreifen (25,26) befestigt ist; wobei die flexiblen, länglichen Aktivierungselemente (C1,C2,C3,C3') ein erstes flexibles, längliches Element (C1) aufweisen, das stromabwärts der Übertragungsanordnung (11) um ein jeweiliges kreisförmiges Element (402), welches einstückig mit einem ersten bewegbaren Arm (202) des End-Effektors (2) ausgebildet ist, festgelegt ist und das stromaufwärts der Übertragungsanordnung (11) zwei erste Abzweige trägt, die jeweils mit einem betreffenden der freien Enden abschließen; und die beiden ersten Abzweige ein erstes Mal um und zwischen eine erste bzw. einer ersten Riemenscheibe (13a) der ersten Reihe von Riemenscheiben (13a-c) und eine erste bzw. einer ersten Riemenscheine (7a) der zweiten Reihe von Riemenscheiben (7a-d) und ein zweites Mal um und zwischen die erste bzw. der ersten Riemenscheibe (7a) der zweiten Reihe von Riemenscheiben (7a-d) und eine erste bzw. einer ersten Riemenscheine (8a) der dritten Reihe von Riemenscheiben (8a-d) kreuzweise verlaufen.

2. Instrument nach Anspruch 1, **dadurch gekennzeichnet, dass** die flexiblen, länglichen Aktivierungselemente (C1,C2,C3,C3') ein zweites flexibles, längliches Element (C2) aufweisen, das stromabwärts der Übertragungsanordnung (11) um ein jeweiliges kreisförmiges Element (302), welches einstückig mit einem zweiten bewegbaren Arm (202) des EndEffektors (2) ausgebildet ist, festgelegt ist und das stromaufwärts der Übertragungsanordnung (11) zwei zweite Abzweige trägt, die jeweils mit einem betreffenden der freien Enden abschließen; und die beiden zweiten Abzweige ein erstes Mal um und zwischen eine zweite bzw. einer zweiten Riemenscheibe (13c) der ersten Reihe von Riemenscheiben (13a-c) und eine zweite bzw. einer zweiten Riemenscheine (7d) der zweiten Reihe von Riemenscheiben (7a-d) und ein zweites Mal um und zwischen die zweite bzw. der zweiten Riemenscheibe (7d) der zweiten Reihe von Riemenscheiben (7a-d) und eine zweite bzw. einer zweiten Riemenscheine (8d) der dritten Reihe von Riemenscheiben (8a-d) kreuzweise verlaufen.

3. Instrument nach Anspruch 1 und 2, **dadurch gekennzeichnet, dass** die ersten und zweiten flexiblen, länglichen Elemente (C1,C2) auf den End-Effektor (2) eine Gierbewegung (Y) relativ zu einem Drehstift (6) ausüben können, um den sich die Arme (102,202) des End-Effektors (2) drehen können.

4. Instrument nach Anspruch 1, **dadurch gekennzeichnet, dass** die flexiblen, länglichen Aktivierungselemente (C1,C2,C3,C3') ein Paar dritter flexibler, länglicher Elemente (C3,C3') aufweisen, die stromabwärts der Übertragungsanordnung (11) mit dem vorderen Teil des Gelenkteils (301) verbunden sind und die stromaufwärts der Übertragungsanordnung (11) mit jeweiligen freien Enden versehen sind, welche mit einem jeweiligen Aktuator verbunden sind, wobei das Paar dritter flexibler, länglicher Elemente (C3,C3') in der Lage ist, mittels des betreffenden Aktuators eine Kippbewegung (P) des End-Effektors (2) relativ zu dem distalen Teil (101) der Welle durchzuführen.

5. Instrument nach Anspruch 4, **dadurch gekennzeichnet, dass** die beiden dritten flexiblen, länglichen Elemente (C3,C3') ein erstes Mal um und zwischen eine dritte bzw. einer dritten Riemenscheibe (13b) der ersten Reihe von Riemenscheiben (13a-13c) und eine dritte bzw. einer dritten Riemenscheine (7b) der zweiten Reihe von Riemenscheiben (7a-d) und ein zweites Mal um eine dritte bzw. einer dritten Riemenscheine (8b) der dritten Reihe von Riemenscheiben (8a-d) kreuzweise verlaufen, wobei die Kippbewegung (P) relativ zu dem dritten Stift (10) durchgeführt wird, der sich seinerseits entsprechend einer eingestellten geometrischen Beziehung um den zweiten Stift (9) dreht.

6. Instrument nach Anspruch 1, **dadurch gekennzeichnet, dass** die Übertragungsvorrichtung (11) ein Paar flexibler, länglicher Halteelemente (C4,C4') aufweist, um der Übertragungsvorrichtung (11) Halt zu geben und die Kinetik und die Dynamik der Kippbewegung (P) des End-Effektors (2) relativ zu dem distalen Teil (101) der Welle (1) zu definieren; wobei die flexiblen, länglichen Halteelemente (C4,C4') jeweilige Rückenden, die mit im hinteren Teil des Gelenkteils (301) angeordneten Spannvorrichtungen (33,34) zusammenwirken, und jeweilige Vorderenden aufweisen, die an dem vorderen Teil des Gelenkteils (301) befestigt sind, und zwar mittels jeweiliger Befestigungselemente (35, 36), die abnehmbar in jeweilige am vorderen Teil des Gelenkteils (301) ausgebildete Sitze (37,38) eingeführt sind

7. Instrument nach Anspruch 6, **dadurch gekennzeichnet, dass** die flexiblen, länglichen Halteelemente (C4,C4') um und zwischen eine vierte bzw. einer vierten Riemenscheibe (7c) der zweiten Reihe von Riemenscheiben (7a-7d) und eine vierte bzw. einer vierten Riemenscheine (8c) der dritten Reihe von Riemenscheiben (8a-8d) kreuzweise verlaufen.

8. Instrument nach Anspruch 1, **dadurch gekennzeichnet, dass** das Instrument ein in der Zwischenverbindungs-Halterung (3) positioniertes Verbindungs- und Löse-System zum Verbinden und Lösen der flexiblen, länglichen Aktivierungselemente (C1,C2,C3,C3') und zum Verbinden und Lösen der Zwischenverbindungs-Halterung (3) mit bzw. von der Welle (1) aufweist, wobei das Verbindungs- und Löse-System mit einem ersten Gleitteil (42,42') versehen ist, das in einem betreffenden ersten Sitz (40,40') gleiten kann, der in dem proximalen Teil (201) der Welle (1) ausgebildet ist und mittels eines betreffenden Verbindungselements (43,43') mit einem zweiten Gleitteil (46,46') zusammengreifen kann, das in einem betreffenden zweiten Sitz (44,44') gleiten kann, der in der Zwischenverbindungs-Halterung (3) ausgebildet ist.

9. Instrument nach Anspruch 8, **dadurch gekennzeichnet, dass** erste elastische Vorspannvorrichtungen (39,39') für die Abzweige des ersten flexiblen, länglichen Elements (C1) in dem ersten Sitz (40,40') des proximalen Teils (101) der Welle (1) positioniert sind.

10. Instrument nach Anspruch 8, **dadurch gekennzeichnet, dass** zweite elastische Vorspannvorrichtungen (47,47') für die Abzweige des ersten flexiblen, länglichen Elements (C1) in dem zweiten Sitz (44,44') der Zwischenverbindungs-Halterung (3) positioniert sind.

11. Instrument nach Anspruch 1, **dadurch gekennzeichnet, dass** das Instrument Messvorrichtungen (41,41') zum Messen der Spannung jedes Abzweigs der ersten und zweiten flexiblen, länglichen Elemente (C1,C2) oder jedes dritten flexiblen, länglichen Elements (C3,C3') aufweist.

12. Instrument nach Anspruch 10 und 11, **dadurch gekennzeichnet, dass** die Messvorrichtungen (41,41') entlang des Verbindungs- und LöseSystems für jeden Abzweig der ersten und zweiten flexiblen, länglichen Elemente (C1) oder für jedes dritte flexible, längliche Element (C3, C3') angeordnet sind.

13. Instrument nach Anspruch 1, **dadurch gekennzeichnet, dass** das Instrument mindestens einen Identifikationsring (12) aufweist.

14. Instrument nach Anspruch 1, **dadurch gekennzeichnet, dass** das Instrument ein auf einem DataMatrix-Label basierendes Erkennungs- und Identifikationssystem aufweist, das in jedem Teil des Instruments positioniert werden kann und eine Reihe von Daten enthalten kann, wie z.B. die seriellen Identifikationsdaten des Instruments, seine Kalibrierungsdaten und andere.

15. Instrument nach Anspruch 1, **dadurch gekennzeichnet, dass** das Instrument ein Erkennungssystem (49,50,51,52) zum automatischen Erkennen der korrekten Einführung des proximalen Teils (201) der Welle (1) in die Zwischenverbindungs-Halterung (3) aufweist, wobei das System auf einer mechanischen Verbindung auf Seiten des Instruments basiert, die entsprechend der Zwischenverbindungs-Halterung (3) ein elektrisches oder optisches Signal oder ein auf einer Kombination der genannten Typen basierendes Signal aktiviert.

16. Instrument nach Anspruch 1, **dadurch gekennzeichnet, dass** jeder der bewegbaren Arme (102,202) des End-Effektors (2) mit einer elektrischen Verbindung (27,28) versehen ist, mittels derer er mit einem Standard-Konnektor eines entsprechenden medizinischen Geräts verbunden werden kann.

17. Instrument nach Anspruch 1, **dadurch gekennzeichnet, dass** das Instrument ein Versatz-Untersetzungssystem für jedes der flexiblen, länglichen Aktivierungselemente (C1,C2,C3,C3') aufweist, wobei das Versatz-Untersetzungssystem Drehelemente (P1-P1', P2-P2', P3-P3') aufweist, um welche die flexiblen, länglichen Aktivierungselemente (C1,C2,C3,C3') gelegt sind; wobei das Versatz-Untersetzungssystem mindestens eine fünfte Riemenscheibe (P1,P1'), die an einem bewegbaren Teil (46) des Instruments befestigt ist, und mindestens eine sechste Riemenscheibe (P2,P2') aufweist, die die an einem festgelegten Teil (57) des Instruments befestigt ist.

18. Instrument nach Anspruch 17 in Abhängigkeit von Anspruch 8, **dadurch gekennzeichnet, dass** die fünfte Riemenscheibe (P1,P1') mittels eines ersten Drehstifts (55) mit dem zweiten Gleitteil (46,46') verbunden ist und die sechste Riemenscheibe (P2,P2') mittels eines zweiten Drehstifts (56) mit dem festgelegten Teil (57) verbunden ist.

19. Instrument nach Anspruch 17, **dadurch gekennzeichnet, dass** die Drehelemente (P1-P1', P2-P2', P3-P3') derart ausgebildet sind, dass die Abzweige der flexiblen, länglichen Aktivierungselemente (C1,C2,C3,C3') parallel zueinander gehalten werden, so dass sich eine lineare Gesetzmäßigkeit des untersetzten Versatzes ergibt.

## Revendications

1. Outil chirurgical robotique, comprenant : un arbre (1) portant une partie distale (101) et une partie proximale (201) ; un poignet articulé (301) avec au moins un degré de liberté relativement au dit arbre (1) ; un effecteur terminal (2) qui est monté sur la partie avant du poignet (301) et est doté de bras mobiles (102, 202) ; un support d'interface (3), qui est raccordé à ladite partie proximale (201) et est raccordable à un système robotique de commande et de vérification de l'outil ; et un ensemble de transmission (11) positionné dans ledit poignet articulé (301) et comprenant une pluralité de poulies (13a-c, 7a-d, 8a-d) positionnées en séquence et une pluralité d'éléments d'activation flexibles et allongés (C1, C2, C3, C3') passant autour des poulies (13a-13c, 7a-7d, 8a-8d) pour permettre le mouvement du poignet (301) en fonction dudit au moins un degré de liberté et/ou le mouvement d'au moins l'un desdits bras mobiles (102, 202) ; chacun desdits éléments d'activation flexibles et allongés (C1, C2, C3, C3') coopérant, en aval dudit ensemble de transmission (11), avec ledit effecteur terminal (2) et avec les bras mobiles relatifs (102, 202), étant doté, en amont dudit ensemble de transmission (11), d'au moins une paire d'extrémités libres raccordables à deux actionneurs (23, 24) de telle manière que lesdits actionneurs (23, 24) peuvent activer lesdits éléments d'activation flexibles et allongés (C1, C2, C3, C3') indépendamment les uns des autres et de manière agoniste/antagoniste pour réaliser ledit mouvement selon ledit au moins un degré de liberté, l'outil étant **caractérisé en ce que** le poignet a une partie arrière raccordée à ladite partie distale (101) et une partie avant raccordée de manière mobile à la partie arrière au moyen de deux brides de raccordement latérales (25, 26) pour fournir au poignet (301) ledit au moins un degré de liberté ; les éléments d'activation allongés (C1, C2, C3, C3') sont croisés autour d'au moins une paire de poulies (13a-13c, 7a-7d, 8a-8d) d'une manière qui découple les axes de rotation des poulies autour desquels ces éléments allongés se croisent ; ladite pluralité de poulies (13a-c, 7a-d, 8a-d) comprend au moins trois séries de poulies (13a13c, 7a-7d, 8a-8d) disposées pour pivoter librement sur trois broches rotatives respectives (14, 9, 10), substantiellement parallèles les unes aux autres ; lesdites broches (14, 9, 10) comprennent une première broche (14) fixée à ladite partie arrière du poignet (301) pour la rotation d'une première série de poulies (13a-13c), une seconde broche (9) fixée aux premières extrémités des deux brides de raccordement latérales (25, 26) pour la rotation d'une seconde série de poulies (7a, 7d), et une troisième broche (10) fixée aux secondes extrémités des deux brides de raccordement latérales (25, 26) pour la rotation de la troisième série de poulies (8a-8d) ; lesdits éléments d'activation flexibles et allongés (C1, C2, C3, C3') comprennent un premier élément flexible et allongé (C1) fixé, en aval de l'ensemble de transmission (11), autour d'un élément circulaire respectif (402) formant partie intégrante d'un premier bras mobile (202) de l'effecteur terminal (2) et portant, en amont de l'ensemble de transmission (11), deux premières branches se terminant, chacune, avec une extrémité libre respective desdites extrémités libres ; et les deux premières branches sont croisées une première fois autour de et entre une première poulie (13a) de ladite première série de poulies (13a-13c) et une première poulie (7a) de ladite seconde série de poulies (7a-7d) et une seconde fois autour de et entre la première poulie (7a) de la seconde série de poulies (7a-7d) et une première poulie (8a) de ladite troisième série de poulies (8a-8d).

2. Outil selon la revendication 1, **caractérisé en ce que** lesdits éléments d'activation flexibles et allongés (C1, C2, C3, C3') comprennent un second élément flexible et allongé (C2) fixe, en aval de l'ensemble de transmission (11), autour d'un élément circulaire respectif (302) formant partie intégrante d'un second bras mobile (202) de l'effecteur terminal (2) et portant, en amont dudit ensemble de transmission (11), deux secondes branches se terminant, chacune, avec une extrémité libre respective desdites extrémités libres ; les deux secondes branches sont croisées une première fois autour de et entre une seconde poulie (13c) de ladite première série de poulies (13a-13c) et une seconde poulie (7d) de ladite seconde série de poulie (7a-7d) et une seconde fois autour de et entre la seconde poulie (7d) de la seconde série de poulies (7a-7d) et une seconde poulie (8d) de ladite troisième série de poulies (8a-8d).

3. Outil selon les revendications 1 et 2, **caractérisé en ce que** lesdits premiers et seconds éléments flexibles et allongés (C1, C2) peuvent transmettre à l'effecteur terminal (2) un mouvement de lacet (Y) relativement à une broche rotative (6), autour de laquelle lesdits bras (102, 202) de l'effecteur terminal (2) peuvent pivoter.

4. Outil selon la revendication 1, **caractérisé en ce que** lesdits éléments d'activation flexibles et allongés (C1, C2, C3, C3') comprennent une paire de troisièmes éléments flexibles et allongés (C3, C3') qui sont raccordés, en aval de l'ensemble de transmission (11), à ladite partie avant du poignet (301) et sont dotés, en amont de l'ensemble de transmission (11), d'une extrémité libre respective raccordée à un actionneur relatif, ladite paire de troisièmes éléments flexibles et allongés (C3, C3') étant capable de réaliser, au moyen de l'actionneur relatif, un mouvement de d'inclinaison (P) de l'effecteur terminal (2) relativement à la partie distale (101) de l'arbre.

5. Outil selon la revendication 4, **caractérisé en ce que** ladite paire de troisièmes éléments flexibles et allongés (C3, C3') sont croisés une première fois autour de et entre une troisième poulie (13b) de ladite première série de poulies (13a-13c) et une troisième poulie (7b) de ladite seconde série de poulies (7a-7d) et une seconde fois autour d'une troisième poulie (8b) de ladite troisième série de poulies (8a-8d), ledit mouvement d'inclinaison (P) étant réalisé relativement à ladite troisième broche (10), qui pivote à son tour autour de ladite seconde broche (9) en fonction d'une relation géométrique définie.

6. Outil selon la revendication 1, **caractérisé en ce que** ledit ensemble de transmission (11) comprend une paire d'éléments de support flexibles et allongés (C4, C4') pour doter l'ensemble de transmission (11) d'un support et définir la cinématique et la dynamique du mouvement d'inclinaison (P) de l'effecteur terminal (2) relativement à la partie distale (101) de l'arbre (1) ; lesdits éléments de support flexibles et allongés (C4, C4') comprenant des extrémités arrières respectives raccordées à des moyens de mise en tension (33, 34) hébergés dans ladite partie arrière du poignet (301) et les extrémités avant respectives fixées à ladite partie avant du poignet (301) au moyen desdits éléments de fixation respectifs (35, 36) insérés de manière amovible dans des logements respectifs (37, 38) formés sur la partie avant du poignet (301).

7. Outil selon la revendication 6, **caractérisé en ce que** lesdits éléments de support flexibles et allongés (C4, C4') sont croisés autour d'une et entre une quatrième poulie (7c) de ladite seconde série de poulies (7a-7d) et une quatrième poulie (8c) de ladite troisième série de poulies (8a-8d).

8. Outil selon la revendication 1, **caractérisée en ce qu'**il comprend un système de raccordement et de débranchement positionné dans ledit support d'interface (3) pour raccorder et débrancher lesdits éléments d'activation flexibles et allongés (C1, C2, C3, C3') et pour raccorder et débrancher le support d'interface (3) à l'arbre (1) et de l'arbre (1) ; le système de raccordement et de débranchement étant doté d'une première coulisse (42, 42'), qui peut coulisser dans un premier logement relatif (40, 40') formé dans la partie proximale (201) de l'arbre (1) et peut se mettre en prise, au moyen d'un élément de raccordement relatif (43, 43'), avec une seconde coulisse (46, 46') qui peut coulisser dans un second logement relatif (44, 44') formé dans le support d'interface (3).

9. Outil selon la revendication 8, **caractérisé en ce qu'**un premier moyen de précontrainte élastique (39, 39') pour lesdites branches dudit premier élément flexible et allongé (C1), est positionné dans ledit premier logement (40, 40') de la partie proximale (101) de l'arbre (1).

10. Outil selon la revendication 8, **caractérisé en ce qu'**un second moyen de précontrainte élastique (47, 47') pour lesdites branches dudit premier élément flexible et allongé (C1), est positionné dans ledit second logement (44, 44') du support d'interface (3).

11. Outil selon la revendication 1, **caractérisé en ce qu'**il comprend un moyen de mesure (41, 41') pour mesurer la tension de chaque branche desdits premier et second éléments flexibles et allongés (C1, C2) ou de chacun des troisièmes éléments flexibles et allongés (C3, C3').

12. Outil selon les revendications 10 et 11, **caractérisé en ce que** ledit moyen de mesure (41, 41') est agencé le long dudit système de raccordement et de débranchement pour chaque branche desdits premier et second éléments flexibles et allongés (C1) ou pour chaque troisième élément flexible et allongé (C3, C3').

13. Outil selon la revendication 1, **caractérisé en ce qu'**il comprend au moins un collier d'identification (12).

14. Outil selon la revendication 1, **caractérisé en ce qu'**il comprend un système de reconnaissance et d'identification basé sur une étiquette DataMatrix, qui doit être positionnée sur une partie quelconque de l'outil et peut contenir une série de données, telles que les données d'identification de série de l'outil, les données d'étalonnage de celui-ci et autres.

15. Outil selon la revendication 1, **caractérisé en ce qu'**il comprend un système de reconnaissance (49, 50, 50, 52) pour automatiquement reconnaître l'insertion correcte de la partie proximale (201) de l'arbre (1) dans le support d'interface (3), ledit système étant basé sur un raccord mécanique sur la partie de l'outil, qui, en fonction du support d'interface (3), active un signal électrique ou optique ou un signal basé sur une combinaison desdits types.

16. Outil selon la revendication 1, **caractérisée en ce que** chacun des bras mobiles (102, 202) de l'effecteur final (2) est doté d'un raccord électrique (27, 28), au moyen duquel il peut être raccordé à un raccord standard d'un appareil médical adapté.

17. Outil selon la revendication 1, **caractérisé en ce qu'**il comprend un système de démultiplication de déplacement pour chacun des éléments d'activation flexibles et allongés (C1, C2, C3, C3'), ledit système de démultiplication de déplacement comprenant des éléments rotatifs (P1-P1', P2-P2', P3-P3') autour desquels lesdits éléments d'activation flexibles et allongés (C1, C2, C3, C3') sont enroulés ; ledit système de démultiplication de déplacement comprenant au moins une cinquième poulie (P1, P1') fixée à une partie mobile (46) de l'outil et au moins une sixième poulie (P2, P2') raccordée à une partie fixe (57) de l'outil.

18. Outil selon la revendication 17 lorsqu'il dépend de la revendication 8, **caractérisé en ce que** ladite cinquième poulie (P1, P1') est raccordée à ladite seconde coulisse (46, 46') au moyen d'une première broche de rotation (55) et ladite sixième poulie (P2, P2') est raccordée à la partie fixe (57) au moyen d'une seconde broche de rotation (56).

19. Outil selon la revendication 17, **caractérisé en ce que** lesdits éléments rotatifs (P1-P1', P2-P2', P3-P3') sont réalisés de manière à maintenir les branches des éléments d'activation flexibles et allongés (C1, C2, C3, C3') parallèles les unes aux autres, permettant ainsi d'obtenir une loi linéaire de déplacement démultiplié.
